(19) Europäisches Patentamt
European Patent Office
Office européen des brevets

(11) **EP 4 458 353 A1**

(12) **EUROPEAN PATENT APPLICATION**

(43) Date of publication:
**06.11.2024 Bulletin 2024/45**

(21) Application number: **23171603.6**

(22) Date of filing: **04.05.2023**

(51) International Patent Classification (IPC):
**A61K 31/352** (2006.01)   **A61K 38/47** (2006.01)
**A61P 1/02** (2006.01)   **A61K 8/49** (2006.01)
**A61K 8/66** (2006.01)   **A61K 8/97** (2017.01)
**A61Q 11/00** (2006.01)

(52) Cooperative Patent Classification (CPC):
(C-Sets available)
**A61Q 11/00; A61K 8/498; A61K 8/66;**
**A61K 8/9789; A61K 31/352; A61K 38/47;**
**A61P 1/02;** A61K 2300/00   (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **SkyLab AG**
**1066 Epalinges (CH)**

(72) Inventors:
• **Belous, Elena Yur'evna**
**121309 MOSCOW (RU)**

• **Yarovaya, Alina Olegovna**
**109518 MOSCOW (RU)**

(74) Representative: **Glawe, Delfs, Moll**
**Partnerschaft mbB von**
**Patent- und Rechtsanwälten**
**Postfach 13 03 91**
**20103 Hamburg (DE)**

Remarks:
The references to the drawing no. 1 is deemed to be deleted (Rule 56(4)(6) EPC).

(54) **ANTICARIOGENIC SYNERGETIC COMPOSITION FOR ORAL CARE**

(57)   The invention is related to the combination of guava leaf extract, naringenin, and enzyme, which is selected from lysozyme, labiase or combination thereof, to be used in the dental or oral care product.

Determination of lysozyme activity in the presence or in the absence of naringenin and/or guava leaf extract in the medium.

Fig. 1

**EP 4 458 353 A1**

(52) Cooperative Patent Classification (CPC): (Cont.)

    C-Sets
    A61K 2300/00, A61K 31/352, A61K 38/47

**Description**

FIELD OF THE INVENTION

The invention is related to the field of cosmetology, dentistry, and hygiene, specifically to the dental care products and oral care products, which provide a high level of resistance to microbial proliferation and favorably impact the degree of the oral mucosa hydration. The composition cane be used as a health or therapeutic product in human and veterinary medicine.

BACKGROUND OF THE INVENTION

Despite incomprehensible efforts made by the global health systems for the greatest coverage of the population with the services of dental clinics and for the wide presentation of various dental care and oral care products in a global market, the decay (dental caries) of permanent teeth remains one of the most prevalent health problems.

Two bacterial species: *Streptococcus mutans* and *Streptococcus sobrinus* have been identified as principal microorganisms triggering the development of dental caries (Mathur and Dhillon, 2018). In the event of inadequate oral care, excessive consumption of sweet product or for other reasons the bacteria of these strains gain significant selective advantages over the rest oral microbiome and proceed to intensive proliferation. The adhesion of these organisms on the tooth surface leads to biofilm (dental plaque) formation, which protects the bacterial colonies from aggressive effects of the human immune system components, antibacterial agents, and other unfavorable factors (Sims et al, 2020; Lemos et al, 2019; Yue et al, 2018; Pleszczyńska et al, 2016; Bhagavathy et al, 2019). *S. mutans,* as the other microbes colonizing the tooth surface use to adhesion mechanisms for biofilm formation, namely, nonspecific and sugar-dependent mechanisms (Lemos et al, 2019; Yue et al, 2018). Non-specific mechanism is implemented through hydrophobic and hydrophilic interactions and the hydrogen bond formation between the superficial structures of bacteria and pellicular (the layer composed of saliva proteins and crevicular fluid on teeth). Sugar-dependent adhesion is conditioned by non-soluble polysaccharide glucan, which has branched structure and is synthesized by bacterial enzymes glycosyl transferases (GTFs) (Lemos et al, 2019; Yue et al, 2018; Cai et al, 2018). Glycan facilitates the streptococci attachment to the tooth surface and also the formation of the aggregates made of streptococci and other bacteria. The life processes of cariogenic species involve the cleavage of readily fermented carbohydrates to lactic acid, which causes focal demineralization and destruction of enamel leading to formation of deep tooth decay cavities (Mathur and Dhillon, 2018; Al-Ansari et al, 2021; Cai et al, 2018; Bhagavathy et al, 2019).

Thus, preventive measures aimed on minimization of the risk of caries development consist primarily in reduction of *S. mutans* and *S. sobrinus* bacterial population in the oral cavity and in prevention of biofilm formation on the tooth surface.

In the claimed invention aimed on control of *S. mutans* and *S. sobrinus* and on destruction of the biofilms formed by the mentioned species two biologically active components have been chosen: naringenin and the lytic enzyme labiase and/or lysozyme. The authors also showed that guava used as a supplementary component can potentiate the effects of the above agents.

Naringenin is a flavonoid exhibiting anti-biofilm, antibacterial, antioxidative, anti-inflammatory and also antitumorogenic properties (Wilcox et al, 1999; Yue et al, 2018). Yue et al. have comprehensively studied the mechanisms of naringenin and showed the capacity for switching off the expression of genes *gtfB* and *gtfC* encoding glycosyl transferase in *S. mutans* (Yue et al, 2018). Consequently, this microorganism loses the capacity for producing the above enzyme and, hence, for synthesizing the glucans, which are essential for sugar-dependent mechanism of adhesion to the tooth surface and for biofilm formation. In addition, naringenin n demonstrated unfavorable effects at the stage of non-specific adhesion of bacteria.

Some authors reported high antibacterial activity of naringenin against *S. mutans:* Minimum Inhibitory Concentration (MIC) of naringenin against this agent ranges from 100 to 200 micrograms/mL ($\mu$g/mL). Naringenin at concentration 200 $\mu$g/mL virtually completely inhibited the biofilm formation.

The advantages of using naringenin include easy availability and simple production of naringenin from the extracts of plant genus Citrus, more often from grapefruit (*Citrus paradisi*) and bigarade (*Citrus aurantium*).

Lytic enzyme lysozyme and/or labiase, which exhibit the biocidal effect manifested in deterioration of the bacterial cell wall, is the second component of the composition.

Lysozyme is a hydrolase enzyme, capable of cleaving $\beta$-$(1{\rightarrow}4)$-glycosidic bond in peptidoglycan molecules, which are present in the cell wall of Gram-positive bacteria (Pleszczyńska et al, 2016). Furthermore, the experiments showed that lysozyme can protect the oral cavity against harmful effects of microorganisms through cation-dependent activation of autolysins, inhibition of bacterial adhesion to the tooth surface and inhibition of glucose metabolism thus leading to lower synthesis of the acids, which attack the enamel (Tenovuo, 2002; Niwa et al, 2005; Pleszczyńska et al, 2016). Lysozyme is commonly used as an active component to control cariogenic bacteria *S. mutans* in the toothpastes, mouth rinses, chewing gums, hydration gels and sprays of such manufacturers as Biotene, BioXtra, Oralbalance and ZendiumSaliva (Tenovuo, 2002; Pleszczynska et al, 2016). It was established that supplementation of these products with lysozyme leads to resolution of dry mouth in the patients with xerostomia and improvement of natural protection of the organism from caries. Antibacterial activity of lysozyme can be enhanced if lysozyme is used in combination with lactoferrin and peroxidase (Pleszczynska et al, 2016).

The lysozyme advantage consists in potential long-term maintenance of activity in the oral care products (Tenovuo, 2002).

Though it is reported in the literature that naringenin can inhibit lysozyme, the authors unexpectedly found synergistic effect of these components. For instance, *Ashrafi et al.* showed that naringenin inhibits lysozyme enzymatic activity and has affinity to the lysozyme active center. Based on the findings of comprehensive study the authors of the study showed that interaction between naringenin and lysozyme leads to modification of conformation and considerably decreased activity of the latter (Ashrafi et al., 2022). Nevertheless, the authors of the present invention found that naringenin and lysozyme exhibit synergistic antibacterial activity.

Labiase is an enzyme having N-acetylglucoseaminidase (disruption of bacterial wall synthesis) and muramidase (lysis of peptidoglycan layer of the cell wall) activities (Ohbuchi K. Et al., 2001). It was shown that labiase is capable of quick and efficient lysis if the cell walls of many Gram-positive bacteria including *S. mutans* (Tenovuo, 2002; Niwa et al, 2005).

High efficacy of antibacterial effect against the lysozyme-resistant strains is one of labiase advantages (Niwa et al., 2005). However, up to now labiase was used only for the cell wall lysis with the purpose of further DNA and RNA isolation (Oana et al., 2009; Ishii et al., 2017).

Guava (*Psidium guajava*) leaf extract contains a broad range of biologically active substances, such as alkaloids, flavonoids, polyphenols, phenols, tannins, saponins, glycosides, and sterols (Garode and Waghode, 2014; Bhagavathy et al, 2019; Shaheena S. et al, 2019). These substances exhibit marked antibacterial activity against many Gram-positive and Gram-negative bacteria (Nayak N., et al, 2019). In particular, tannins dissolve the lipids in the bacterial cell wall thus leading to osmotic rupture of the wall and the death of the whole cell (Netrusov, Kotova, 2018; Bhagavathy et al, 2019). Polyphenols inhibit the activity of some bacterial virulence factors including lipopolysaccharides, various bacterial enzymes, etc. (Bhagavathy et al, 2019; Chen et al, 2005). In 2014 *Garode* and *Waghode* extracted bioactive components from guava leaves with the use of such solvents as acetone, methanol, benzene, petroleum ether, chloroform, and ethanol and then used agar diffusion method to assess the biocidal effect of the extracts in *S. mutans* strain model (Garode and Waghode, 2014). These authors showed that acetonic, methanolic and petroleum ether solutions of the guava extracts had the antibacterial activity comparable to antibiotic ampicillin activity.

Anti-biofilm effect of the methanolic extract of guava leaves was proved in the study of 2019 (Bhagavathy et al, 2019). It appeared that some components of the extract can interact with glucosyl transferase (GTF) active center being the competitor to substrate (saccharose) and inhibiting the synthesis of the principal biofilm component, i.e., glucan.

In the opinion of majority of authors, the advantages of guava leaf extract include: readily available raw material, easy extraction process, and environmental friendliness. The authors of the present invention performed several experiments, which showed that guava can further potentiate antibacterial activity of the composition containing naringenin and lysozyme.

The above-mentioned components were used separately in various oral care products and dental care products. However, the synergistic effect thereof was not previously disclosed.

The toothpaste containing lysozyme and velvet plant (*Gynura procumbens* (Lour.) Merr) extract was previously described (see patent CN105534827B; published on 23.02.2018; IPC A61K8/97, A61K8/66, A61Q11/00 etc.). The toothpaste contains lysozyme and the velvet plant extract at weight ratio of 1:10 ~ 10:1. The manufacturers reported the toothpaste capacity for good cleaning the teeth and oral cavity from the pathogenic bacteria without emergence of microorganisms resistant to the active components of toothpaste.

The disadvantage of the above toothpaste is that it contains only one active component, lysozyme, which is targeted on inhibition of bacteria proliferation and the biofilm disruption, while the velvet plant extract has anti-inflammatory, hemostatic, and the wound healing activities. By now at least three mechanisms of overcoming the lysozyme biocidal effect have been identified in bacteria (Ragland and Criss, 2017). Thus accumulation of lysozyme-resistant bacteria in the oral cavity will eventually lead to decrease in efficacy of the proposed toothpaste.

The toothpaste R.O.C.S. "Balsam For Gums" is manufactured by The Commercial and Industrial Group of Companies "DRC". The composition of this product includes the asp tanning agents, sodium hydrocarbonate, xylitol (6%), benzoic acid, populin glycosides, salicin glycosides, enzyme salicase, flavonoids naringenin and sacuranten, coumarin, and also saturate fatty acids: palmitic acid, behenic acid, lignoceric acid, and cerotic acid. However, this toothpaste was developed for prophylaxis of inflammatory diseases of oral cavity rather than for caries prevention, because the listed components exhibit mainly anti-inflammatory, decongestive, and healing effects.

"Guava Herbal Toothpaste" containing guava extract is manufactured by Beautecrets (Thailand). The components in composition of this toothpaste include the extracts of tropic guava *Psidium guajava* and great morinda *Morinda*

*citrifolia*, and also the clove *Syzygium aromaticum* leaf oil and peppermint *Mentha piperíta* oil, which are distinguished for anti-inflammatory, anesthetic, antiviral, and antimicrobial effects, while the peppermint is also used as a deodorant. However, this product is presented by the manufacturer as a refreshing toothpaste because of the too slight antimicrobial effect.

Another whitening herbal toothpaste containing guava and clove extracts "HERBAL CLOVE TOOTHPASTE" manufactured by 5 Star Cosmetic (Thailand) is also known. The components of this toothpaste are as follows: calcites, borneol, menthol, dodecyl sulphate, Stebelius leaf extract, guava leaf extract, camphor, bamboo salt, and clove extract. It should be noted that the search of original information on the product, the manufacturer, and composition thereof is a rather challenging task. As follows from description of the product, the above components have antiseptic effect, remove dental plaque and protect from caries (https://www.japshop.ru/good/otbelivayuschie-udalenie-kamnya-naleta-10003485/100081-25-/?ymclid=16014590748368498010500004), this information is consistent with numerous scientific investigations of guava leaf extract. However, it is a common knowledge that antibacterial effect directly depends on concentration and the method of extraction of guava leaves (Nair and Chanda, 2007; Garode and Waghode, 2014). There are no indications on particular guava extract and the percentage of the active component in the composition of the toothpaste, in this connection there are some doubts on the claimed properties of this component. The component "CLOVE" in the name of the toothpaste suggests that the clove extract is the main component of the toothpaste and is responsible for the health benefits of the concerned product. However, there no indications of percentage of the clove extract in the composition of the product, while this is an important measure determining antibacterial activity (Kaur & Chandrul, 2017). Thus, the indicated active components of the above toothpaste probably have predominantly marketing function.

Another known composition contains polyphenols/flavonoids and the derivatives thereof for treatment or prevention the dental caries, dental plaque formation, gingivitis, periodontitis, candidiasis, denture stomatitis, and fungal infectious diseases (see US20140314686A1, published on 23.10.2014; IPC A61K8/66, A61Q11/00, A61Q17/005, A61P1/02 etc.). The compositions are presented as various combinations of aglyconic flavones, proanthocyanidins, dihydroflavonols, flavonols and flavanols including naringenin, which are added in therapeutically effective concentrations. The compositions introduced into the oral cavity inhibit the activity of glucosyl transferase on the teeth surface and also suppress the growth and proliferation of microorganisms (in particular, glucosyl transferase-producing microbes). Nevertheless, this composition does not comprise the enzymes lysozyme and/or labiase.

Another known antimicrobial combination composition contains one or more glycerol derivatives, which are selected from the group consisting of 3-[(2-ethylhexyl)oxy]-1,2-propanediol, 3-[(n-octyl)oxy]-1,2-[propanediol and 3-[(2-octyl)oxy]-1,2-propanediol], and one or more bicyclic compounds, which are selected from the group consisting of: naringenin, quercetin, hesperitin and eriodictyol (see patent CA3104962A1 published on 09.01.2020; IPC A61Q17/00 etc.). The composition is designed for addition into the toothpastes, mouth rinses and other hygienic products. This composition is also free of lysozyme and/or labiase.

The limitation of the above compositions arises from the fact that they contain the components having the same antibacterial mechanism as naringenin has, thus providing the conditions for development of bacterial resistance to the product in the event of long-term use.

The closest prior art to the claimed invention in terms of the technical substance are the compositions of additives containing lysozyme and the plant extracts (see patent CN110237241A; published on 17.09.2019; IPC A61Q11/00, A61K38/47, A61K8/66, A61P31/04 etc.). The plant extracts exhibit primarily anti-inflammatory and wound healing activities and are presented by *Centella asiatica* (Gotu Kola P.E) extract, *Polygonum japonicum* Meissn. root extract, Baikal scullcap (*Scutellaria baicalensis*) extract, common licorice (*Glycyrrhiza glabra*) extract, German pellitory (*Chamomilla recutita*) bloom extract, the tea *Camellia sinensis* leaf extract, and dwarf rosemary (*Rosmarinus officinalis*) extract, or the extract of respective varieties. These extracts contain various alkaloids, essential oils, flavones, polysaccharides, organic acids, tannins, etc. as active components, which have different antibacterial potency. Some flavones exhibit the properties similar to naringenin. In particular, apigenin found in the extracts of German pellitory, dwarf rosemary, and common licorice are produced in the plants as a result of naringenin oxidation by the enzyme flavanone synthase.

The disadvantage of such compositions is undue use of the great amount of plant extracts exhibiting similar biological activities. Complexity of the compositions inevitably complicates the manufacturing process and increase financial and time expenditures. Furthermore, standardization of chemical composition of the plant extract is a process-complicated task, and the use thereof as the oral care additives, as follows from the description, provides low bacteriostatic effect.

The present invention overcomes all above disadvantages though combination of a few components (from two to four) having essentially different mechanisms of biocidal activity in one composition, thus enabling to achieve synergetic increase in antimicrobial and anticariogenic efficacy thereof. Overall, two strategies of the effect implemented by the claimed composition can be specified:

- Eradication of biofilms, which is achieved due to the targeted naringenin exposure and additionally due to effect of guava leaf extract on synthesis of glucans, which are an important matrix component. In particular, naringenin disrupts glycosyl transferase synthesis at a gene level thus inhibiting *S. mutans* adhesion to the dental enamel. Guava leaf extract binds to the active center of the enzyme glycosyl transferase and also inhibits the glucane synthesis.

- Decrease in bacterial population due to lytic enzymes (lysozyme and/or labiase), which kill the bacterial cells by destruction of the cell wall.

Since the mechanisms of the composition components and not species-specific, the antibacterial and anti-biofilm activities thereof are relevant also to *S. sobrinus,* which is closely related to *S. mutans.* There is every reason to suppose that the claimed composition does not induce bacterial resistance, and this is an obvious advantage, which enables regular use of the composition as the oral care product.

DISCLOSURE OF THE INVENTION

The invention is related to a combination of naringenin and an enzyme selected from lysozyme, labiase or combination thereof for the use in the dental care product and/or oral care product.

A combination of the invention can be distinguished by that the combination additionally contains the guava leaf extract.

A combination of the invention can be distinguished by that a guava leaf extract is guava leaf CO2 extract.

A combination of the invention can be distinguished by that a guava leaf extract *is Psidium guajava* leaf extract.

A combination of the invention can be distinguished by that a guava leaf extract is *Psidium guajava* leaf CO2 extract.

A combination of the invention can be distinguished by that naringenin is obtained from the plant source.

A combination of the invention can be distinguished by that a guava leaf extract, naringenin, and enzyme are contained at ratio (1.00-170.73) : (1.00-235.77) : (0.16-365.85), respectively.

A combination of the invention can be distinguished by that a combination is a composition.

The invention is related to the dental care product and/or to the oral care product containing a combination of the invention.

A product of the invention can be distinguished by that the % wt of the components is as follows:

| | |
|---|---|
| guava leaf extract | 0.0615-10.5. |
| naringenin | 0.0615-14.5. |
| enzyme | 0.0096-22.5 . |

A product of the invention, can be distinguished by that a product is a toothpaste or a mouth rinse.

The invention is related to the use of combination of the invention to control *Streptococcus mutans* and/or *Streptococcus sobrinus* on the teeth surface and/or in the oral cavity.

The invention is related to the use of dental care product and/or oral care product to control *Streptococcus mutans* and/or *Streptococcus sobrinus* on the teeth surface and/or in the oral cavity.

The technical results achieved due to invention are unexpected anti-biofilm and antibacterial synergistic effect, favorable impact on the oral cavity hydration, and also effects and actions understandable for those skilled in the art, either disclosed or directly following from this specification, in particular, from experimental examples.

BRIEF DESCRIPTION OF THE DRAWINGS

Fig. 1 depicts the findings obtained from the assessment of Streptococcus sobrinus viability (in singlicate) by resazurin test.
Fig. 2 depicts the findings obtained from the assessment of synergistic ani-biofilm effect.

DETAILED DESCRIPTION OF THE INVENTION

The authors unexpectedly found that the use of naringenin and enzyme (selected from lysozyme, labiase or combination thereof) in the form of composition provides pronounced synergistic effect manifested in considerable enhancement of anti-biofilm and antibacterial effects and also in improved hydration of the oral cavity.

Additional beneficial effects and actions understandable for those skilled in the art are disclosed throughout the text of this document or directly follow from specification of the invention, in particular, from the obtained experimental data.

In the first aspect the invention the invention is related to the combination of naringenin and the enzyme selected from lysozyme, labiase, or combination thereof. Said combination can be useful in the dental care product and/or oral care product.

Said combination can be supplemented also with guava leaf extract. Such combination can be useful in a dental care product and/or oral care product.

Guava can be used as fruit, bark of leaves of *Psidium guajava.* Naringenin can be obtained from any plant source.

The combination of the invention can be distinguished by that guava leaf extract is guava CO2 extract, for example, *Psidium guajava* CO2 extract.

In the combination a guava extract, naringenin and enzyme are taken at weight ratio (1,00 - 170,73) : (1,00 - 235,77) : (0,16 - 365,85), respectively, wherein said ranges include the boundary numerical values and all numerical values within the range, as if each of such numerical values is expressly indicated. Put this another way, such record discloses the boundary numerical values, all values between the boundary values, and any ranges of values made up from said boundary values and each numerical value within the range.

Combination of the invention can be distinguished by that the combination is a composition. Therefore, the combination can be not related to the mixture of substances and cover, for instance, concomitant and non-fixed administration of the components, while composition is a particular case of combination is related to the mixture of the components.

The invention is directed also to the dental care product and/or oral care product containing the combination of the invention, for instance, such as toothpaste or mouth rinse, with the following composition of the components (% wt):

| | |
|---|---|
| guava leaf extract | 0.0615-10.5, |
| naringenin | 0.0615-14.5, |
| enzyme | 0.0096-22.5. |

In addition, the invention covers the use of the combination of invention or the dental and/or oral care product of the invention to control *S. mutans* and/or *S. sobrinus* on the teeth surface and/or in the oral cavity.

EXAMPLES

**Group of examples No 1 - 3**. Antibacterial activity of the substance or a composition of substances can be assessed in terms of minimum inhibitory concentration (MIC) in accordance with recommendations of MUK (Methodological Guidelines) 4.2.1890-04 and Leclercq et al., 2013 and from the growth pattern in liquid nutritive medium.

Reference strains *S. mutans* and *S. Sobrinus* are used as bacterial models.

Guava leaf extract (Psidium guajava Leaf Extract), which was used in all examples, was obtained by modifier-enhanced supercritical CO2 extraction. The extract content of the finished sample of raw material was at least 50% (the other components include water (up to 100%), glycerol (up to 30%), sodium benzoate (0.01%). Naringenin for all examples was isolated from Grapefruit extract. Naringenin concentration in the finished raw material was 98%. Labiase used in all tests was a pure enzyme beta-N-acetyl-D-glucosaminidase isolated from *Streptomyces sp.* Lysozyme used in all tests was 99% anhydrous lysozyme hydrochloride isolated from the egg yolks.

**Example №1.** The following procedure is used to determine MIC of separate composition components, i.e. naringenin, guava leaf $CO_2$ extract, enzymes lysozyme and labiase:

1) Prepare liquid nutritive medium LB composed of: $H_2O_{dist}$ 1L, tripton 1.0 %; yeast extract 0.5 %; NaCl 0.5 % . Adjust pH is to 8.0. Sterilize the medium by autoclaving for 20 minutes at 121 °C. Then add sterile glucose solution to final concentration 1% and bovine serum to 5 %.

2) Using microbiological loop prepare the suspensions (at concentration $1.5 \cdot 10^8$ CFU/mL) from 24 hour agar cultures of *S. mutans* and *S. sobrinus* in the sterile buffered saline (BS) under visual control with McFarland standard 0.5. Dilute the obtained suspension in sterile nutritive medium LB 1:100 to obtain final cell concentration $1.5 \cdot 10^6$ CFU/mL.

3) Add 100 µL of the test substance at concentration 2-fold exceeding maximum concentration into well 1 of sterile 96-well microtitration plate (Medpolymer, RF) and make serial two-fold dilutions with sterile distilled water to minimum concentration. Then add into each well 100 µL of prepared inoculum with the bacterial suspension density at $10^6$ CFU in 1 mL of sterile nutritive medium LB, thus diluting two-fold the test compounds.

4) Prepare the following controls: control of the culture growth (3 wells with liquid nutritive medium LB diluted 2-fold with sterile distilled water, with addition of bacterial culture, and without addition of test substances); control for the nutritive medium purity (3 wells with liquid nutritive medium LB diluted 2-fold with sterile distilled water without addition of bacterial culture); control of solvents (3 wells with liquid nutritive medium LB diluted 2-fold with sterile distilled water with addition of test substance or test composition and without addition of bacterial culture).

5) Incubate the plates agitating in orbital shaker at 37 °C for 24 hours.

6) Assess the viability of planktonic bacterial cell culture of bacterial using resazurin test [Taneja N.K., Tyagi J.S. Resazurin reduction assays for screening of anti-tubercular compounds against dormant and actively growing Mycobacterium tuberculosis, Mycobacterium bovis BCG and Myco-bacterium smegmatis. JAntimicrob Chemother. 2007; 60(2): 288-293]. For this purpose, after completion of incubation add 10 µL of 0.01 % aqueous resazurin solution (Sigma-Aldrich, USA) into each well of the plate wait for 5 min for the color development in solution under the same conditions.

7) The test is based on colorimetric assay of cell metabolic activity: in the presence of living cells blue-colored resazurin is reduced to pink-red resorufin. Developing pink color of resazurin solution indicates that the tested concentration is not sufficient for complete inhibition of metabolic activity of bacteria. For this reason, the first well where the solution does not turn pink is regarded as minimum inhibitory concentration of the test substance (MIC). Since the serial dilution method with 2-fold dilution steps is used for MIC determination, the difference between the adjacent dilution is considered as non-significant.

8) The following concentrations were tested: naringenin and guava leaf extract at concentrations 1.6, 0.8, 0.4, 0.2, 0.1, 0.05 mg/mL; lysozyme and labiase at concentrations 4.0, 2.0, 1.0, 0.5, 0.25, 0.125 mg/mL. Well-known antibacterial components were used as controls for antibacterial activity: chlorohexidine bigluconate at concentrations 64, 32, 16, 8, 4 µg/mL; zinc gluconate at concentrations 5.0, 2.5, 1.25, 0.6, 0.3, 0.15 mg/mL.

MIC was defined as the median of the inhibitory concentration values determined in 5 independent biological replicates of the experiment.

In Table 1 the results of the test components assessment for antibacterial activity are summarized.

Table 1 - Minimal concentration (**MIC**) of the substances inhibiting the growth of bacterial species *Streptococcus mutans* and *Streptococcus sobrinus*

| Bacterial culture | MIC value in individual replicate | | | | | Median value |
|---|---|---|---|---|---|---|
| | Replicate 1 | Replicate 2 | Replicate 3 | Replicate 4 | Replicate 5 | |
| **Naringenin**, mg/mL | | | | | | |
| *S. mutans* | >1.6 | >1.6 | 0.8 | 0.4 | 0.8 | 0.8 |
| *S. sobrinus* | >1.6 | >1.6 | >1.6 | >1.6 | >1.6 | >1.6 |
| **Guava leaf extract**, mg/mL | | | | | | |
| *S. mutans* | >1.6 | >1.6 | >1.6 | >1.6 | >1.6 | >1.6 |
| *S. sobrinus* | >1.6 | >1.6 | >1.6 | >1.6 | >1.6 | >1.6 |
| **Lysozyme**, mg/mL | | | | | | |
| *S. mutans* | 4.0 | 4.0 | 4.0 | 4.0 | 4.0 | 4.0 |
| *S. sobrinus* | 4.0 | >4.0 | 4.0 | >4.0 | 4.0 | 4.0 |
| **Labiase**, mg/mL | | | | | | |
| *S. mutans* | >4.0 | >4.0 | >4.0 | >4.0 | >4.0 | >4.0 |
| *S. sobrinus* | >4.0 | >4.0 | >4.0 | >4.0 | >4.0 | >4.0 |
| **Zinc gluconate,** mg/mL | | | | | | |
| *S. mutans* | 0.6 | 0.6 | 0.3 | 0.3 | 0.3 | 0.3 |
| *S. sobrinus* | 5.0 | 5.0 | 5.0 | 5.0 | 5.0 | 5.0 |
| **Chlorohexidine bigluconate**, µg/mL | | | | | | |
| *S. mutans* | 8.0 | 8.0 | 8.0 | 8.0 | 8.0 | 8.0 |
| *S. sobrinus* | 4.0 | 4.0 | 4.0 | 8.0 | 4.0 | 4.0 |

As is evident from Table 1, test strain *S. mutans* is more sensitive to antibacterial effect of zinc gluconate compared to *S. sobrinus.* However, this strain much higher resistance to chlorohexidine bigluconate.

Naringenin and lysozyme at concentrations 0.8 and 4.0 mg/L, respectively, successfully inhibited the growth of strain *S. mutans.*

Lysozyme was the only component, which at concentration 4.0 mg/L demonstrated the capacity for growth inhibition in the experiment with *S. sobrinus.*

Thus, two closely related cariogenic species *S. mutans* and *S. sobrinus* showed different level of sensitivity to various antibacterial agents. Based on these findings the inclusion of substances showing different sets of biocidal mechanisms into the antibacterial composition is relevant and reasonable.

**Example № 2**. Synergistic antibacterial effect of naringenin, the guava leaf extract and a lytic enzyme, specifically lysozyme and/or labiase, in the composition was assessed by MIC determination. All steps of MIC determination were similar to those described in Example 1, but 100 μL of the composition "naringenin + guava leaf extract + enzyme" were added into the wells of the plate instead of individual substances. Naringenin and guava leaf extract were used in composition at constant concentration 0.8 mg/mL. The concentrations of lysozyme and labiase in composition varied: 1.0, 0.5 and 0.25 mg/mL. The final composition containing naringenin + guava leaf extract + enzyme was also diluted 1:1, 1:2, and 1:4, and MIC was determined for the resultant dilutions.

MIC was defined as the median of the inhibitory concentration values determined in 4 independent biological replicates of the experiment. The synergism of the obtained mixture against cariogenic bacteria was determined by assessment of the enzyme concentration, which was required to inhibit bacterial growth, when used as a monocomponent and in combination with guava leaf extract and naringenin at different dilution of final composition.

Table 2 shows the results of assessment of the components used in composition "naringenin (0.8 mg/mL) + guava leaf extract (0.8 mg/mL) + enzyme (1.0 - 0.25 mg/mL)" for antibacterial activity against cariogenic bacteria *S. mutans* and *S. sobrinus,* when composition was tested at concentration 100%, 50% and 25%.

Table 2 - Differences in lysozyme or labiase minimum concentrations (**MIC**) inhibiting the growth of *Streptococcus mutans* and *Streptococcus sobrinus,* in composition "naringenin + guava leaf extract + enzyme"

| Bacterial culture | MIC decrease in individual replicate, fold | | | | |
| --- | --- | --- | --- | --- | --- |
| | Replicate 1 | Replicate 2 | Replicate 3 | Replicate 4 | Median value |
| **Lysozyme** | | | | | |
| *S. mutans* | 4 | 4 | 2 | 4 | 4 |
| *S. sobrinus* | 16 | 32 | 32 | 8 | 24 |
| **Labiase** | | | | | |
| *S. mutans* | no | no | no | no | no |
| *S. sobrinus* | 32 | 32 | 16 | 16 | 32 |

In the composition all components were taken at concentrations much lower compared to previously determined MIC for both bacterial strains (Table 1).

It is evident from Table 2 that in the presence of naringenin 0.8 mg/mL and guava leaf extract 0.8 mg/mL lysozyme MICs for *S. mutans* and *S. sobrinus* showed 4-fold decrease and 24-fold derease, respectively. Labiase MIC labiase for *S. sobrinus* decreased 32-fold, while the labiase antibacterial activity for *S. mutans* left unchanged.

Therefore, it was concluded that antibacterial effect of the composition "naringenin + guava leaf extract + enzyme" for both cariogenic bacteria has increased due to synergistic effect reflected by decrease in MIC of lytic enzymes in the systems compared to MIC of individual agents (Table 1).

**Example № 3**. Antibacterial activity of naringenin, guava leaf extract and lysozyme was assessed from the growth pattern of the reference strain *Streptococcus mutans* No 22081 (UNU Collection under I. I. Mechnikov Research Institute if Vaccines and sera) in liquid nutritive medium according to the procedure:

1) Prepare the heart-brain broth (DM106D, Mast Group Ltd., U.K.) and Columbian agar supplemented with defibrinated blood (OOO "GEM" Cat. No BP-023) in accordance with the manufacturer's instruction.

2) Using microbiological loop prepare the suspension (at concentration $1.5 \cdot 10^8$ CFU/mL) of 24 hour agar culture of *S. mutans* in the sterile buffered saline (BS) under visual control with McFarland standard 0.5. Dilute the obtained suspension in BS to the concentration $10^6$ CFU/mL.

3) Into the wells of 96-well microtitration plate (Medpolymer, RF) add 240 μL of heart-brain broth, 30 μL of *S. mutans*

suspension at concentration $10^6$ CFU/mL, and 30 μL of the test substance taken from naringenin, guava leaf extract or lysozyme at test concentration. Prepare the composition of naringenin 0.33 mg/mL, lysozyme 0.33 mg/mL, and guava leaf extract 0.33 mg/mL.

4) Put the plate with the samples into the incubator with 5% $CO_2$ atmosphere, and incubate at 37°C for 5 h on shaking apparatus.

5) After the 5 h incubation reinoculate the agar nutritive medium with the planktonic *S. mutans* culture to count CFU per 1 mL of medium.

6) Perform optical density (OD) measurements using multimodal microplate reader at the wavelength 620 nm before the incubation ($OD_0$) and then after the culture growth for 5 h $OD_{t=5}$). When calculating distract mean optical density obtained at baseline measurement from the mean value of resultant optical density to balance intrinsic color of the test samples ($OD_{t=5}$ - $OD_0$).

Calculate the inhibition level (%) of *S. mutans* culture growth from the equation:

$$\%OD = OD_{substance}\ (OD_{t=5} - OD_0)\ /\ OD_{control}\ (OD_{t=5} - OD_0) \times 100 - 100,\ (\%) \qquad (2)$$

7) Measure each concentration of the test substance in 3 replicates; measure the control sample in 12 replicates.

The results of assessment of antibacterial activity of naringenin, guava leaf extract, and lysozyme in liquid nutritive medium against *S. mutans* are shown in Table 3.

Table 3 - The effect on growth of S. mutans planktonic culture in liquid nutritive medium.

| Active ingredient | $\Sigma$ CFU, mL | % of CFU obtained for control |
|---|---|---|
| Control | $9 \times 10^8$ | 100 |
| Lysozyme (1 mg/mL) | $4.5 \times 10^7$ | 5 |
| Naringenin (1 mg/mL) | $4.3 \times 10^7$ | 4.8 |
| Guava extract (1 mg/mL) | $4.6 \times 10^7$ | 5.1 |
| Composition (1 mg/mL) | $2.1 \times 10^7$ | 2.3 |

As is evident from Table 3, all test substances considerably affected the growth of *S. mutans* planktonic culture in liquid nutritive medium. The composition containing lysozyme, guava leaf extract, and naringenin at final concentration 1 mg/mL exhibited the greatest inhibitory effect.

The composition showed synergistic effect on the growth of *S. mutans* planktonic culture compared to effects of the individual components.

**Group of examples No 4** - **6.** Anti-biofilm activity of the substance or of the composition of substances was assessed from minimum biofilm inhibitory concentration (MBIC) and from the rate of biofilm formation on hydroxyapatite disks.

Reference strains *Streptococcus mutans* and *S. Sobrinus* were used as the bacterial models.

**Example No 4.** MBIC as the measure of the substance effect on the bacterial biofilm is equal to minimum concentration inhibiting the biofilm formation or disruption of already formed biofilm during 24 hour incubation.

MBIC of individual components of the composition, specifically, naringenin, guava leaf $CO_2$ extract, and enzymes lysozyme and labiase can be determined according to the procedure:

1) All stages of nutritive medium preparation, preparation and incubation of bacterial cultures in 96-well microtiter plates with the test composition of the substances added into nutritive medium were as described in Example 1.

2) All controls for the culture growth, for purity of nutritive medium and for solvents were as described in Example 1.

3) Incubate the plates at 37 °C for 24 ± 4 h without the use the orbital shaker.

4) Stain the bacterial biofilm accumulated on bottom of the plate wells with crystal violet. For this purpose remove the planktonic culture from the plate wells after 24 h incubation and wash with two portions of phosphate-buffered saline (PBS, pH 7.4) to remove remaining planktonic culture. Then dry the plate for 12-15 hours. Add 100 μL of 0.5% crystal violet solution (Sigma-Aldrich, USA), dissolved in 96% ethanol followed by incubation for 20 minutes. After removal of crystal violet solution from the wells wash the plate with 3-4 portions of PBS. After drying add 1 mL of 96% ethanol into the wells to elute the crystal violet, which has bound to the biofilm.

5) Shake the plate on the orbital shaker for 2 minutes at 37 °C and then measure optical density (OD) at the wavelength of 550nm using multimodal microplate reader against the blank (the well, where the sterile medium LB was incubated and then exposed to all staining manipulations). Since the serial dilution method with 2-fold dilution steps is used for MBIC determination, the difference between the adjacent dilution is considered as non-significant, and MBIC is defined as the median value calculated for 5 independent biological replicates of the experiment.

6) The following concentrations were tested: naringenin and guava leaf extract at concentrations 1.6, 0.8, 0.4, 0.2, 0.1, 0.05 mg/mL; lysozyme and labiase at concentrations 4.0, 2.0, 1.0, 0.5, 0.25, 0.125 mg/mL. Well-known antibacterial components were used as controls: chlorohexidine bigluconate at concentrations 64, 32, 16, 8, 4 $\mu$g/mL; zinc gluconate at concetrations 5.0, 2.5, 1.25, 0.6, 0.3, 0.15 mg/mL.

In Table 4 the results of the test components assessment for anti-biofilm activity in relation to the biofilms produced by cariogenic bacterial species are summarized.

Table 4 - Minimum biofilm inhibitor concentration (**MBIC**) of the substances for the bacterial species *Streptococcus mutans* and *Streptococcus sobrinus*

| Bacterial culture | MBIC in indicvdual replicates | | | | | Median value |
|---|---|---|---|---|---|---|
| | Replicate 1 | Replicate 2 | Replicate 3 | Replicate 4 | Replicate 5 | |
| **Naringenin**, mg/mL | | | | | | |
| *S. mutans* | 0.8 | 0.8 | 0.8 | 0.4 | 0.8 | 0.8 |
| *S. sobrinus* | >1.6 | >1.6 | >1.6 | >1.6 | >1.6 | >1.6 |
| **Guava leaf extract**, mg/mL | | | | | | |
| *S. mutans* | >1.6 | >1.6 | >1.6 | >1.6 | >1.6 | >1.6 |
| *S. sobrinus* | >1.6 | >1.6 | >1.6 | >1.6 | >1.6 | >1.6 |
| **Lysozyme**, mg/mL | | | | | | |
| *S. mutans* | 4.0 | 4.0 | 2.0 | 4.0 | 2.0 | 4.0 |
| *S. sobrinus* | 4.0 | 2.0 | 2.0 | 4.0 | 4.0 | 4.0 |
| **Labiase**, mg/mL | | | | | | |
| *S. mutans* | >4.0 | >4.0 | >4.0 | >4.0 | >4.0 | >4.0 |
| *S. sobrinus* | >4.0 | >4.0 | >4.0 | >4.0 | >4.0 | >4.0 |
| **Zinc gluconate**, mg/mL | | | | | | |
| *S. mutans* | 1.25 | 1.25 | 0.6 | 0.6 | 0.6 | 0.6 |
| *S. sobrinus* | 5.0 | 5.0 | 2.5 | 2.5 | 2.5 | 2.5 |
| **Chlorohexidine bigluconate**, $\mu$g/mL | | | | | | |
| *S. mutans* | 8.0 | 8.0 | 8.0 | 8.0 | 4.0 | 8.0 |
| *S. sobrinus* | 4.0 | 4.0 | 8.0 | 4.0 | 4.0 | 4.0 |

As is evident from Table 4, the test strain *S. mutans* produces less biofilm in the presence of zinc gluconate compared to *S. sobrinus.* However, this strain is much more resistant to chlorohexidine bigluconate.

Naringenin and lysozyme at concentrations 0.8 and 4.0 mg/L, respectively, successfully inhibited the biofilm formation by strain *S. mutans.*

Lysozyme at concentration 4.0 mg/L was also able to prevent the biofilm formation by the strain *S. sobrinus.*

Thus, two closely related cariogenic species *S. mutans* and *S. sobrinus* showed different level of sensitivity to different mechanisms of anti-biofilm effects of the test agents.

**Example № 5.** Synergistic anti-biofilm effect of naringenin, guava leaf extract and lytic enzyme, specifically, lysozyme and/or labiase in the composition is assessed through determination of MBIC. All stages of the method used for MBIC determination are similar to that described in Example 4, except that in the plate well 100 $\mu$L of the composition "naringenin + guava leaf extract + enzyme" is added instead of individual substances. Naringenin and guava leaf extract was used in the composition at constant concentration 0.8 mg/mL. The concentrations of lysozyme and labiase in composition varied: 1.0, 0.5 and 0.25 mg/mL. The final composition containing naringenin + guava leaf extract + enzyme was additionally diluted 1:1, 1:2, and 1:4, and MBIC of the resultant mixture was determined.

MBIC was defined as the median of the inhibitory concentration values determined in 4 independent biological replicates of the experiment. The synergism of the obtained mixture against cariogenic bacteria was determined by assessment of the enzyme concentration, which was required to inhibit bacterial biofilms, when used as a monocomponent and in combination with guava leaf extract and naringenin at different dilution of final composition.

Table 5 summarizes the results of assessment of the components used in the composition "naringenin (0.8 mg/mL) + guava leaf extract (0.8 mg/mL) + enzyme (1.0 - 0.25 mg/mL)" for synergistic anti-biofilm activity against cariogenic bacteria *S. mutans* and *S. sobrinus.*

Table 5 - Differences in lysozyme or labiase minimum biofilm inhibitory concentrations **(MBIC)** for *Streptococcus mutans* and *Streptococcus sobrinus,* in composition "naringenin + guava leaf extract + enzyme"

| Bacterial culture | MBIC decrease in an individual replicatee, times | | | | |
|---|---|---|---|---|---|
| | Replicate 1 | Replicate 2 | Replicate 3 | Replicate 4 | Median value |
| **Lysozyme** | | | | | |
| *S. mutans* | 32 | 16 | 32 | 32 | 32 |
| *S. sobrinus* | 16 | 8 | 16 | 16 | 16 |
| **Labiase** | | | | | |
| *S. mutans* | 32 | 32 | 16 | 32 | 32 |
| *S. sobrinus* | 16 | 16 | 8 | 8 | 12 |

As is evident from Table 5, the combination of naringenin 0.8 mg/mL and naringenin and guava leaf extract 0.8 mg/mL provided 32-fold decrease in lysozyme and labiase inhibitory concentrations for biofilm formation by *S. mutans*; and 16-fold and 12-fold decrease in lysozyme and labiase inhibitory concentrations, respectively, for biofilm formation by *S. sobrinus.*

Therefore, the compositions containing naringenin (0.8 mg/mL), guava leaf extract (0.8 mg/mL) and lytic enzyme (1.0-0.25 mg/mL)" selected from lysozyme and labiase, provided enhanced anti-biofilm effect against both cariogenic species of genus *Streptococcus.* Strong synergistic activity of the system was observed, because the required effect was achieved with considerably lower concentrations of both naringenin and guava leaf extract, and labiase and lysozyme, when used as the composition compared to MBIC of individual substances (Table 4).

Example № 6. Anti-biofilm activity of individual substances selected from naringenin, guava leaf extract, and lysozyme, or the composition thereof, is assessed from the rate of biofilm formation on hydroxyapatite disks, with the use of the following procedure:

1) Prepare the heart-brain broth (DM106D, Mast Group Ltd., U.K.) and Columbian agar supplemented with defibrinated blood (OOO "GEM" Cat. No BP-023) in accordance with the manufacturer's instruction
2) All stages of preparation of the bacterial culture suspensions were similar to those described in Example 1.
3) Prepare saliva, which is required for biofilm formation and provides the bacteria adhesion to the surface of hydroxyapatite disks. Collect the saliva from 15 healthy, non-smoking, adult volunteers, which did not take antibacterial during the last 3 months, into sterile, cooled tube not earlier than 1 hour after the food intake or tooth brushing. Centrifuge the collected saliva at 12 000 rpm for 30 minutes at 4°C. Harvest the supernatant into the clean tube and subject to pasteurization at 65°C for 30 minutes followed by one more centrifugation.

The efficacy of saliva pasteurization is assessed from direct inoculation on Columbian blood agar. The plates are incubated under aerobic and anaerobic conditions at 37°C for 72 hours. The saliva is considered sterile if no growth of bacterial and fungal colonies is observed within 72 hours of observation.

Store the saliva at temperature -80 °C before the use.

4) To form the salivary film on the surface of hydroxyapatite disk put three sterile disks (sterilization by autoclaving at 132 °C for 20 min) in each of 9 Petri dishes 40 mm in diameter abd incubate in 3 mL of the pasteurized saliva at room temperature for 4 hours with gentle shaking on orbital shaker. Then remove the saliva and add 3 mL of nutritive medium containing 1.2 mL of heart-brain broth; 1.2 mL of pasteurized saliva; 0.6 mL of *S. mutans* suspension at concentration of $10^6$ CFU/mL into the Petri dish. The formation of *S. mutans* biofilm on the disk surface is achieved at 37°C under the atmosphere of 5 % $CO_2$ in the environment, with gentle shaking on orbital shaker for 16 hours.

5) After 16-hour incubation remove old nutritive medium from the dishes and add a fresh portion of heart-brain broth containing *S. mutans* suspension and the pasteurized saliva. Add the active substances selected from naringenin, guava leaf extract and lysozyme or combination thereof to final concentration 0.1 or 1.0 mg/mL. Repeat the procedure of medium replacement twice daily within 4 f and 8 h after the first change.

6) To remove the biofilm formed in the disk surface take the disks away from Petri dishes after the incubation for 2 days and rinse in the large volume of PBS (pH 7.2). Then place the disks into sterile Petri dishes with 3 mL of PBS (pH 7.2) and shake vigorously on the orbital shaker for 30 minutes. Transfer the resultant *S. mutans* suspension into marked sterile tubes and determine CFU/mL by inoculation of the agar nutritive medium.

Table 6 summarizes the results of inoculation with biofilms washed from the disk surfaces after 64-hour incubation in the presence of individual active substances or the composition of substances.

Table 6 - Quantitative comparison of CFU in resuspended *S. mutans* biofilms, formed on the surface of hydroxyapatite disks during 64h incubation in the presence of naringenin, guava leaf extract, lysozyme or composition thereof

| Active substance | $C_{substance}$ | N $_{S.mutans}$ (CFU/mL) | %N $_{Control}$ |
|---|---|---|---|
| Naringenin | 0.1 mg/mL | $2.8 \cdot 10^6$ | 66.7 |
| | 1.0 mg/mL | $2.1 \cdot 10^6$ | 50.0 |
| Guava leaf extract | 0.1 % | $5.7 \cdot 10^5$ | 13.6 |
| | 1.0 % | $5.4 \cdot 10^6$ | 128.6 |
| Lysozyme | 0.1 mg/mL | $3.7 \cdot 10^6$ | 88.1 |
| | 1.0 mg/mL | $2.3 \cdot 10^6$ | 54.8 |
| Composition | 0.1 mg/mL | $6.2 \cdot 10^6$ | 147.2 |
| | 1.0 mg/mL | $1.1 \cdot 10^7$ | 262.0 |
| -* | - | $4.2 \cdot 10^6$ | 100.0 |

Notes. (*) - Control. $C_{substance}$ - concentration of the test substance; N $_{S.mutans}$ - CFU of S. *mutans* on the disk after 5 h incubation in the presence of the substance; %N $_{Control}$ - percentage of *S. mutans* CFU on the disk in control experiment.

In this experiment *S. mutans* biofilm partially disrupted by shaking was used for inoculation. In these conditions every fragment of biofilm will form one *S. mutans* colony on the agar nutritive medium. One should take into consideration that the more active are destructive processes in the biofilm under exposure to active substances during incubation, the more pronounced is destruction of chemical bonds between the components of the biofilm matrix, and the greater number of biofilm fragments is formed during shaking, and, consequently, the number of colony-forming units (CFU) increases.

Table 6 shows that exposure of guava leaf extract at concentration 1.0 mg/mL was associated with marked destruction of the biofilm. However, 10-fold decrease in the active substance concentration in the culture medium resulted in much lower level of destruction.

In addition to anti-biofilm activity, naringenin and lysozyme, showed high antibacterial activity against *S. mutans* (Tables 1, 2 and 3). Perhaps, this provides and explanation of modest CFU/mL values in relation to control. These substances retained their biocidal potential when concentrations decreased to 0.1 mg/mL - the level of viable cells in suspensions compared to Control was 66.7 % and 88.1 %, respectively.

The greatest destruction was observed for *S. mutans* biofilm after exposure to the composition "naringenin + guava leaf extract + lysozyme" at concertation 1.0 mg/mL. The level of colony forming fragments of biofilm reached 262% compared to Control. The composition retains high antibiofilm activity at 10-fold lower concentration due to synergistic effect developed from concomitant impact of several destructive mechanisms.

**Example № 7.** Assessment of impact of the active composition "naringenin + guava leaf extract + enzyme" on hydration (salivation) of the oral cavity.

Equipment:

- Containers for saliva collection, such as Salivette® (Sarstedt Inc., Germany), containing cotton swab without any reagent fix in the special holder in the upper part of the container.

- Analytical balance with an accuracy m $\pm$ 0.0001 r and upper weighting limit at least 300 g;
- Tooth brushes Professional Complete (SPLAT) of intermediate hardness.

The compositions of two toothpastes tested in the experiment are shown in Table 7.

Table 7 - Comparative characteristics of chemical compositions of the toothpastes

| No | Chemical substance | Content in toothpaste (% on a dry weight basis) | |
|---|---|---|---|
| | | without active composition | with active composition |
| 1 | Purified water | Up to 100 | Up to 100 |
| 2 | Sorbitol, 70% | 35.00 | 35.00 |
| 3 | Silicon dioxide | 28.00 | 28.00 |
| 4 | Glycerol | 7.00 | 7.00 |
| 5 | Sodium Koko-sulphate | 1.50 | 1.50 |
| 6 | Sodium carboxymethylcellulose | 0.85 | 0.85 |
| 7 | Potassium nitrate | 2.50 | 2.50 |
| 8 | Sodium hydrocarbonate | 0.40 | 0.40 |
| 9 | Sodium hexametaphosphate | 1.00 | 1.00 |
| 10 | Enzyme subtilisin | 0.01 | 0.01 |
| 11 | Polidon A | 0.30 | 0.30 |
| 12 | Dicalcium phosphate dihydrate$_T$ | 5.00 | 5.00 |
| 13 | Calcium pyrophosphate | 2.00 | 2.00 |
| 14 | Flavoring agent | 1.00 | 1.00 |
| 15 | Menthol | 0.09 | 0.09 |
| 16 | Benzyl alcohol | 0.40 | 0.40 |
| 17 | Sodium lauryl sarcosinate | 5.50 | 5.50 |
| 18 | Grape fruit extract (naringenin) | - | 0.08 |
| 19 | Guava leaf $CO_2$ extract | - | 0.08 |
| 20 | Lysozyme hydrochloride | - | 0.01 |

The test procedure:

1) The containers for saliva collection Salivette® are marked, then weighed three times on the electronic balance, and the mean arithmetic weight of each container is calculated and designated as "Weight 1.1".
2) 10 volunteers are randomly distributed to Group A to test the toothpaste free of the active composition (5 subjects) or Group B to test the toothpaste containing the active composition (5 subjects).
3) All volunteers clean the teeth for 2 minutes using the tooth brush with the sample of test toothpaste (the weight of paste portion ~ 2 mg).
4) Then the swabs are taken from the marked containers and put into the mouth of each subject. Two minutes later the swabs are returned with a forceps into respective containers.
5) Then the containers with collected saliva are weighed thrice, and mean arithmetic value is calculated for three measurements and designated as "Weight 1.2".
6) The weight of collected saliva is calculated by the equation:

$$\text{Saliva weight} = \text{Weight 1.2} - \text{Weight 1.1} \qquad (2).$$

7) The test is repeated twice so that the subjects test the toothpaste samples in random order. Each time the weight of collected saliva is calculated by the equation (2).
8) After completion of the test mean arithmetic weight of saliva collected from each subject during the test of the toothpaste either free of active composition (Group A) or containing the active composition (Group B) is calculated.
9) Mann-Whitney U test (nonparametric analysis) or Student t-test for comparison of unrelated populations (parametric analysis) are used to establish the significant differences between the groups.

The test results are shown in Table 8.

Table 8 - Determination of statistically significant differences in weight of secreted saliva in the subjects who tested two types of the toothpaste

| Subject No. | The weight of secreted saliva during the test in Group: | | The difference in saliva weighs in Groups A and B | Mann-Whitney U-test | p |
|---|---|---|---|---|---|
| | A | B | | | |
| 1 | 2.070 ± 0.015* | 3.150 ± 0.021 * | -1.080 | | |
| 2 | 1.547 ± 0.009 | 1.957 ± 0.010 | -0.410 | | |
| 3 | 0.714 ± 0.005 | 1.607 ± 0.006 | -0.893 | | |
| 4 | 0.675 ± 0.004 | 1.213 ± 0.008 | -0.538 | | |
| 5 | 1.782 ± 0.010 | 3.168 ± 0.017 | -1.386 | 20 | <0.05 |
| 6 | 0.852 ± 0.008 | 1.431 ± 0.005 | -0.579 | | |
| 7 | 0.645 ± 0.002 | 0.783 ± 0.007 | -0.138 | | |
| 8 | 1.103 ± 0.007 | 1.791 ± 0.004 | -0.688 | | |
| 9 | 1.200 ± 0.011 | 2.716 ± 0.003 | -1.516 | | |
| 10 | 1.232 ± 0.015 | 1.759 ± 0.005 | -0.527 | | |

Notes. (*) - mean arithmetic value calculated for three measurements. Group A - the test of the toothpaste without active composition; Group B - the test of the toothpaste containing the active composition . Critical value of Mann-Whitney U-test for prescribed figures of the compared groups = 23.

It is evident from Table 8 that all subjects demonstrated the differences in salivation levels after the use of the toothpastes with or without the active substance composition. It was established that the toothpaste containing the active composition statistically significantly increased the salivation level in the subjects (Group B) compared to the toothpaste without active composition (Group A).

Example № 8. Assessment of lysozyme and naringenin compatibility Potential impact of naringenin on lysozyme activity was assessed through the measurement of lysozyme activity by the method described in the patent RU2294373C2. To determine the lysozyme activity the optical density values obtained for the control wells are subtracted from the optical density values obtained for the wells, which were incubated in the presence of lysozyme. The bacterial cells *Micrococcus lysodeicticus* in in amount corresponding to the optical density 0.6 are mixed with the equal volume of buffer containing lysozyme, naringenin, guava leaf $CO_2$ extract, while no lysozyme is added into the control well. Then the plate is incubated for 30, 60, 120 minutes at 37 degrees, and the optical density is measured. The fall of optical density indicates the lysozyme activity. Then the final density value is subtracted from the initial density value followed by normalization for incubation time and the enzyme concentration in accordance with the procedure described in RU2294373C2.

The Diagram 1 shows that lysozyme activity values remain the same with account for statistical error regardless of addition of naringenin and/or guava extract to the buffered medium. These results demonstrate that naringenin and guava extract neither affect lysozyme nor decrease the lysozyme activity thus supporting the combination of these components for synergistic effect on the cariogenic bacteria and biofilms.

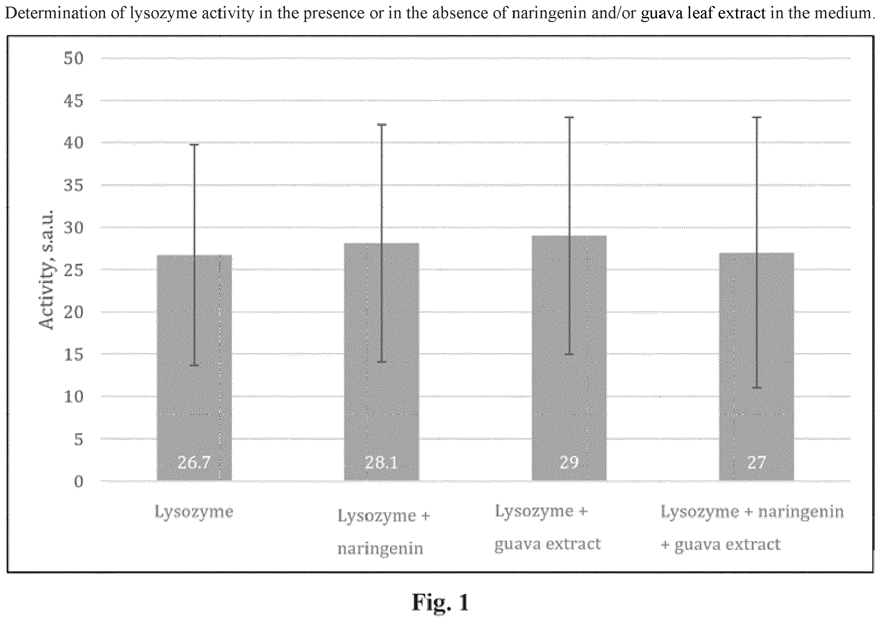

Fig. 1 shows the lysozyme activity values in specific activity units (s.a.u.).

REFERENCES

1. Global Burden of Disease Collaborative Network. Global Burden of Disease Study 2019 (GBD 2019). Seattle: Institute of Health Metrics and Evaluation (IHME); 2020. Available at http://ghdx.healthdata.org/gbd-results-tool.

2. Microbiology: theory and practice. In 2 parts. Part 1: Textbook for Baccalaureate and Master's Course / A. I. Netrusov, I. B. Kotova. - M.: Uright Publishing Company, 2018. - 315 c.-Series: Bachelor and Master. Academical Course.

3. Kuleshiva Svetlana Ivanovna. Determination of antibiotic activity by agar diffusion method // Journal of Scientific Center For Expert Evaluation of Medical Products. 2015. № 3.

4. Al-Ansari, M. M., Al-Dahmash, N. D., & Ranjitsingh, A. J. A. (2021). Synthesis of silver nanoparticles using gum Arabic: Evaluation of its inhibitory action on Streptococcus mutans causing dental caries and endocarditis. Journal of Infection and Public Health, 14(3), 324-330.

5. Bhagavathy, S., Mahendiran, C., & Kanchana, R. (2019). Identification of glucosyl transferase inhibitors from Psidium guajava against Streptococcus mutans in dental caries. Journal of traditional and complementary medicine, 9(2), 124-137.

6. Shaheena S., Chintagunta A.D., Dirisala V.R., Sampath Kumar N.S. Extraction of bioactive compounds from Psidium guajava and their application in dentistry. AMB Express. 2019. V.9, № 1. P. 208. doi: 10.1186/s13568-019-0935-x.

7. Nayak N., Varghese J., Shetty S., Bhat V., Durgekar T., Lobo R., Nayak U.Y., Vishwanath U. Evaluation of a mouthrinse containing guava leaf extract as part of comprehensive oral care regimen- a randomized placebo-controlled clinical trial. BMC Complement Altern Med. 2019. V.19, № 1. P. 327. doi: 10.1186/s12906-019-2745-8.

8. de Soet JJ, van Loveren C, Lammens AJ, Pavicic MJ, Homburg CH, ten Cate JM, et al. Differences in cariogenicity between fresh isolates of Streptococcus sobrinus and Streptococcus mutans . Caries Res. 1991;25:116-22.

9. Okada M, Kawamura M, Oda Y, Yasuda R, Kojima T, Kurihara H. Caries prevalence associated with Streptococcus mutans and Streptococcus sobrinus in Japanese schoolchildren. Int J Paediatr Dent. 2012;22:342-8.

10. Okada M, Soda Y, Hayashi F, Doi T, Suzuki J, Miura K, et al. Longitudinal study of dental caries incidence associated with Streptococcus mutans and Streptococcus sobrinus in pre-school children. J Med Microbiol. 2005;54:661-5.

11. Chen, L., Yang, J., Yu, J., Yao, Z., Sun, L., Shen, Y., & Jin, Q. (2005). VFDB: a reference database for bacterial virulence factors. Nucleic acids research, 33(suppl_1), D325-D328.

12. Garode, A. M., & Waghode, S. M. (2014). Antibacterial activity of guava leaves extracts against S. mutans. Inter J Bioassays, 3(10), 3370-2.

13. Ibrahim, M. S., Ibrahim, A. S., Balhaddad, A. A., Weir, M. D., Lin, N. J., Tay, F. R., ... & Melo, M. A. S. (2019). A novel dental sealant containing dimethylaminohexadecyl methacrylate suppresses the cariogenic pathogenicity of Streptococcus mutans biofilms. International journal of molecular sciences, 20(14), 3491.

14. Ishii, S., Fukui, K., Yokoshima, S. et al. High-throughput Screening of Small Molecule Inhibitors of the Streptococcus Quorum-sensing Signal Pathway. Sci Rep 7, 4029 (2017). https://doi.org/10.1038/s41598-017-03567-2.

15. Jian-Na Cai, Ji-Eun Jung, Min-Ho Lee, Hyeon-Mi Choi, Jae-Gyu Jeon, Sucrose challenges to Streptococcus mutans biofilms and the curve fitting for the biofilm changes, FEMS Microbiology Ecology, Volume 94, Issue 7, July 2018, fiy091, https://doi.org/10.1093/femsec/fiy091.

16. Jun, J.-Y.; Jung, M.-J.; Jeong, I.-H.; Yamazaki, K.; Kawai, Y.; Kim, B.-M. Antimicrobial and Antibiofilm Activities of Sulfated Polysaccharides from Marine Algae against Dental Plaque Bacteria. Mar. Drugs 2018, 16, 301. https://doi.org/10.3390/md16090301.

17. Kassebaum NJ, Bernabé E, Dahiya M, Bhandari B, Murray CJ, Marcenes W. Global burden of untreated caries: a systematic review and metaregression. J Dent Res. 2015 May;94(5):650-8. doi: 10.1177/0022034515573272. Epub 2015 Mar 4. PMID: 25740856.

18. Kaur, D., & Chandrul, K. K. (2017). Syzygium aromaticum L.(Clove): A vital herbal drug used in periodontal disease. Indian Journal of Pharmaceutical and Biological Research, 5(02), 45-51.

19. Lemos, J. A., Palmer, S. R., Zeng, L., Wen, Z. T., Kajfasz, J. K., Freires, I. A., Abranches, J., & Brady, L. J. (2019). The Biology of Streptococcus mutans. Microbiology spectrum, 7(1), 10.1128/microbiolspec.GPP3-0051-2018. https://doi.org/10.1128/microbiolspec.GPP3-0051-2018.

20. Mathur, V.P., Dhillon, J.K. Dental Caries: A Disease Which Needs Attention. Indian J Pediatr 85, 202-206 (2018). https://doi.org/10.1007/s12098-017-2381-6.

21. Nair, R., & Chanda, S. (2007). In-vitro antimicrobial activity of Psidium guajava L. leaf extracts against clinically important pathogenic microbial strains. Brazilian Journal of Microbiology, 38, 452-458.

22. Niwa, T., Kawamura, Y., Katagiri, Y., & Ezaki, T. (2005). Lytic enzyme, labiase for a broad range of Gram-positive bacteria and its application to analyze functional DNA/RNA. Journal of microbiological methods, 61(2), 251-260.

23. Nomura, R., Matayoshi, S., Otsugu, M., Kitamura, T., Teramoto, N., & Nakano, K. (2020). Contribution of severe dental caries induced by Streptococcus mutans to the pathogenicity of infective endocarditis. Infection and immunity, 88(7), e00897-19.

24. Oana, K., Kawakami, Y., Hayashi, T., & Ohnishi, M. (2009). Simple broad-spectrum protocol using labiase for bacterial cell lysis to prepare genomic DNA for pulsed-field gel electrophoresis analysis. Microbiology and immunology, 53(1), 45-48.

25. Philip, N., Suneja, B. & Walsh, L. Beyond Streptococcus mutans: clinical implications of the evolving dental caries aetiological paradigms and its associated microbiome. Br Dent J 224, 219-225 (2018). https://doi.org/10.1038/sj.bdj.2018.81.

26. Pitts, N., Zero, D., Marsh, P. et al. Dental caries. Nat Rev Dis Primers 3, 17030 (2017). https://doi.org/10.1038/nrdp.2017.30.

27. Pleszczynska, Malgorzata; Wiater, Adrian; Bachanek, Teresa; Szczodrak, Janusz (2016). Enzymes in therapy of biofilm-related oral diseases. Biotechnology and Applied Biochemistry, (), n/a-n/a. doi:10.1002/bab.1490

28. Sims Jr, K. R., Maceren, J. P., Liu, Y., Rocha, G. R., Koo, H., & Benoit, D. S. (2020). Dual antibacterial drug-loaded nanoparticles synergistically improve treatment of Streptococcus mutans biofilms. Acta Biomaterialia, 115, 418-431.

29. Schleifer, K. H., & Kandler, O. (1972). Peptidoglycan types of bacterial cell walls and their taxonomic implications. Bacteriological reviews, 36(4), 407-477.

30. Tang, X., Kudo, Y., Baker, J. L., LaBonte, S., Jordan, P. A., McKinnie, S. M., ... & Edlund, A. (2020). Cariogenic Streptococcus mutans produces tetramic acid strain-specific antibiotics that impair commensal colonization. ACS infectious diseases, 6(4), 563-571.

31. Tenovuo, J. (2002). Clinical applications of antimicrobial host proteins lactoperoxidase, lysozyme and lactoferrin in xerostomia: efficacy and safety. Oral diseases, 8(1), 23-29.

32. Valm A. M. The structure of dental plaque microbial communities in the transition from health to dental caries and periodontal disease //Journal of molecular biology. - 2019. - T. 431. - №. 16. - C. 2957-2969.

33. Yue, J., Yang, H., Liu, S., Song, F., Guo, J., & Huang, C. (2018). Influence of naringenin on the biofilm formation of Streptococcus mutans. Journal of dentistry, 76, 24-31.

34. Wilcox, L. J., Borradaile, N. M., & Huff, M. W. (1999). Antiatherogenic properties of naringenin, a citrus flavonoid. Cardiovascular drug reviews, 17(2), 160-178.

**Claims**

1. A combination of

    i) naringenin and
    ii) an enzyme selected from lysozyme, labiase or combination thereof,
    iii) and optionally guava leaf extract,

to be used in a dental care product and/or in an oral care product.

2. The combination of claim 1, wherein said guava leaf extract is the guava leaf CO2 extract.

3. The combination of claim 1, wherein said guava leaf extract is *Psidium guajava* leaf extract.

4. The combination of claim 3, wherein said guava leaf extract is *Psidium guajava* leaf CO2 extract.

5. The combination of any of the preceding claims, wherein said naringenin is obtained from a plant source.

6. The combination of any of the preceding claims, wherein said guava leaf extract, naringenin and enzyme are contained at weigh ratio of (1.00-170.73) : (1.00-235.77) : (0.16-365.85) respectively.

7. A composition comprising the combination of any of the preceding claims.

8. A dental care product and/or an oral care product containing the combination of any of the preceding claims 1.

9. The product of claim 8, wherein the % wt of the components is as follows:

| guava leaf extract | 0.0615-10.5, |
| naringenin | 0.0615-14.5, |
| enzyme | 0.0096-22.5. |

10. The product of claim 9, wherein the product is a toothpaste or a mouth rinse.

11. Use of the combination of any of claims 1 to 7 to control *Streptococcus mutans* and/or *Streptococcus sobrinus* on the teeth surface and/or in the oral cavity.

12. Use of the dental care product and/or the oral care product of any of claims 8 to 10 to control *Streptococcus mutans* and/or *Streptococcus sobrinus* on the teeth surface and/or in the oral cavity.

13. The use of claim 11 or claim 12, wherein said control comprises inhibition of proliferation and/or growth of said *Streptococcus mutans* and/or *Streptococcus sobrinus* on said teeth surface and/or in said oral cavity.

14. Use of the combination of any of claims 1 to 7 or the product of any of claims 8-10 as a dental care product and/or as an oral care product.

15. The combination of any of claims 1 to 7 or the product of any of claims 8-10 for use in the prophylaxis or treatment of caries.

Determination of lysozyme activity in the presence or in the absence of naringenin and/or guava leaf extract in the medium.

Fig. 1

**EUROPEAN SEARCH REPORT**

Europäisches
Patentamt
European
Patent Office
Office européen
des brevets

Application Number

EP 23 17 1603

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 107 582 419 A (ZHANG LEI) 16 January 2018 (2018-01-16) * claims; Examples * ----- | 1-15 | INV. A61K31/352 A61K38/47 A61P1/02 A61K8/49 A61K8/66 A61K8/97 A61Q11/00 |
| A | CN 110 279 609 A (GUANGZHOU KANGYUN BIOLOGICAL TECH CO LTD) 27 September 2019 (2019-09-27) * claims; Examples * ----- | 1-15 | |
| A | AMMAR N ET AL: "FLAVONOIDS AS A POSSIBLE PREVENTIVE OF DENTAL PLAQUE", ARCHIVES OF PHARMACAL RESEARCH (SEOUL), vol. 13, no. 2, 1990, pages 211-213, XP055646346, ISSN: 0253-6269 * page 211, abstract * ----- | 1-15 | |
| A | JIAXI YUE ET AL: "Influence of naringenin on the biofilm formation of Streptococcus mutans", JOURNAL OF DENTISTRY, vol. 76, 1 September 2018 (2018-09-01), pages 24-31, XP055646347, AMSTERDAM, NL ISSN: 0300-5712, DOI: 10.1016/j.jdent.2018.04.013 * page 24, abstract * ----- | 1-15 | TECHNICAL FIELDS SEARCHED (IPC) A61K A61P A61Q |

-/--

The present search report has been drawn up for all claims

| Place of search | Date of completion of the search | Examiner |
|---|---|---|
| Munich | 6 October 2023 | Baurand, Petra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

**EP 23 17 1603**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GUSTAFSSON BENGT E. ET AL: "The Caries Reducing Effect of Naringenin and of Protamine in Hamsters", ACTA ODONTOLOGICA SCANDINAVICA, vol. 16, no. 4, 2 January 1958 (1958-01-02), pages 355-361, XP093089072, NO ISSN: 0001-6357, DOI: 10.3109/00016355809028188 Retrieved from the Internet: URL:http://dx.doi.org/10.3109/000163558090 28188> * page 360, Summary * | 1-15 | |
| | | | **TECHNICAL FIELDS SEARCHED (IPC)** |

The present search report has been drawn up for all claims

| Place of search | Date of completion of the search | Examiner |
|---|---|---|
| Munich | 6 October 2023 | Baurand, Petra |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 23 17 1603

This annex lists the patent family members relating to the patent documents cited in the above-mentioned European search report.
The members are as contained in the European Patent Office EDP file on
The European Patent Office is in no way liable for these particulars which are merely given for the purpose of information.

06-10-2023

| Patent document cited in search report | | Publication date | Patent family member(s) | Publication date |
|---|---|---|---|---|
| CN 107582419 | A | 16-01-2018 | NONE | |
| CN 110279609 | A | 27-09-2019 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 105534827 B
- US 20140314686 A1
- CA 3104962 A1
- CN 110237241 A
- RU 2294373 C2

### Non-patent literature cited in the description

- **TANEJA N.K. ; TYAGI J.S.** Resazurin reduction assays for screening of anti-tubercular compounds against dormant and actively growing Mycobacterium tuberculosis, Mycobacterium bovis BCG and Myco-bacterium smegmatis. *JAntimicrob Chemother,* 2007, vol. 60 (2), 288-293
- Global Burden of Disease Study. Institute of Health Metrics and Evaluation, 2019
- Microbiology: theory and practice. **A. I. NETRUSOV ; I. B. KOTOVA. - M.** Textbook for Baccalaureate and Master's Course. Uright Publishing Company, 2018, 315
- **KULESHIVA SVETLANA IVANOVNA.** Determination of antibiotic activity by agar diffusion method. *Journal of Scientific Center For Expert Evaluation of Medical Products,* 2015
- **AL-ANSARI, M. M. ; AL-DAHMASH, N. D. ; RANJITSINGH, A. J. A.** Synthesis of silver nanoparticles using gum Arabic: Evaluation of its inhibitory action on Streptococcus mutans causing dental caries and endocarditis. *Journal of Infection and Public Health,* 2021, vol. 14 (3), 324-330
- **BHAGAVATHY, S. ; MAHENDIRAN, C. ; KANCHANA, R.** Identification of glucosyl transferase inhibitors from Psidium guajava against Streptococcus mutans in dental caries. *Journal of traditional and complementary medicine,* 2019, vol. 9 (2), 124-137
- **DE SOET JJ ; VAN LOVEREN C ; LAMMENS AJ ; PAVICIC MJ ; HOMBURG CH ; TEN CATE JM et al.** Differences in cariogenicity between fresh isolates of Streptococcus sobrinus and Streptococcus mutans. *Caries Res,* 1991, vol. 25, 116-22
- **OKADA M ; KAWAMURA M ; ODA Y ; YASUDA R ; KOJIMA T ; KURIHARA H.** Caries prevalence associated with Streptococcus mutans and Streptococcus sobrinus in Japanese schoolchildren. *Int J Paediatr Dent,* 2012, vol. 22, 342-8
- **OKADA M ; SODA Y ; HAYASHI F ; DOI T ; SUZUKI J ; MIURA K et al.** Longitudinal study of dental caries incidence associated with Streptococcus mutans and Streptococcus sobrinus in pre-school children. *J Med Microbiol,* 2005, vol. 54, 661-5
- **CHEN, L. ; YANG, J. ; YU, J. ; YAO, Z. ; SUN, L. ; SHEN, Y. ; JIN, Q.** VFDB: a reference database for bacterial virulence factors. *Nucleic acids research,* 2005, vol. 33 (1), D325-D328
- **GARODE, A. M. ; WAGHODE, S. M.** Antibacterial activity of guava leaves extracts against S. mutans. *Inter J Bioassays,* 2014, vol. 3 (10), 3370-2
- **IBRAHIM, M. S. ; IBRAHIM, A. S. ; BALHADDAD, A. A. ; WEIR, M. D. ; LIN, N. J. ; TAY, F. R. ; MELO, M. A. S.** A novel dental sealant containing dimethylaminohexadecyl methacrylate suppresses the cariogenic pathogenicity of Streptococcus mutans biofilms. *International journal of molecular sciences,* 2019, vol. 20 (14), 3491
- **ISHII, S. ; FUKUI, K. ; YOKOSHIMA, S. et al.** High-throughput Screening of Small Molecule Inhibitors of the Streptococcus Quorum-sensing Signal Pathway. *Sci Rep,* 2017, vol. 7, 4029, https://doi.org/10.1038/s41598-017-03567-2
- **JIAN-NA CAI ; JI-EUN JUNG ; MIN-HO LEE ; HYEON-MI CHOI ; JAE-GYU JEON.** Sucrose challenges to Streptococcus mutans biofilms and the curve fitting for the biofilm changes. *FEMS Microbiology Ecology,* July 2018, vol. 94 (7), fiy091, https://doi.org/10.1093/femsec/fiy091
- **JUN, J.-Y. ; JUNG, M.-J. ; JEONG, I.-H. ; YAMAZAKI, K. ; KAWAI, Y. ; KIM, B.-M.** Antimicrobial and Antibiofilm Activities of Sulfated Polysaccharides from Marine Algae against Dental Plaque Bacteria. *Mar. Drugs,* 2018, vol. 16, 301, https://doi.org/10.3390/md16090301
- **KASSEBAUM NJ ; BERNABÉ E ; DAHIYA M ; BHANDARI B ; MURRAY CJ ; MARCENES W.** Global burden of untreated caries: a systematic review and metaregression. *J Dent Res,* 04 March 2015, vol. 94 (5), 650-8
- **KAUR, D. ; CHANDRUL, K. K.** Syzygium aromaticum L.(Clove): A vital herbal drug used in periodontal disease. *Indian Journal of Pharmaceutical and Biological Research,* 2017, vol. 5 (02), 45-51

- **LEMOS, J. A. ; PALMER, S. R. ; ZENG, L. ; WEN, Z. T. ; KAJFASZ, J. K. ; FREIRES, I. A. ; ABRANCHES, J. ; BRADY, L. J.** The Biology of Streptococcus mutans. *Microbiology spectrum,* 2019, vol. 7 (1, https://doi.org/10.1128/microbiolspec.GPP3-0051-2018
- **MATHUR, V.P. ; DHILLON, J.K.** Dental Caries: A Disease Which Needs Attention. *Indian J Pediatr,* 2018, vol. 85, 202-206, https://doi.org/10.1007/s12098-017-2381-6
- **NAIR, R. ; CHANDA, S.** In-vitro antimicrobial activity of Psidium guajava L. leaf extracts against clinically important pathogenic microbial strains. *Brazilian Journal of Microbiology,* 2007, vol. 38, 452-458
- **NIWA, T. ; KAWAMURA, Y. ; KATAGIRI, Y. ; EZA-KI, T.** Lytic enzyme, labiase for a broad range of Gram-positive bacteria and its application to analyze functional DNA/RNA. *Journal of microbiological methods,* 2005, vol. 61 (2), 251-260
- **NOMURA, R. ; MATAYOSHI, S. ; OTSUGU, M. ; KI-TAMURA, T. ; TERAMOTO, N. ; NAKANO, K.** Contribution of severe dental caries induced by Streptococcus mutans to the pathogenicity of infective endocarditis. *Infection and immunity,* 2020, vol. 88 (7), e00897-19
- **OANA, K. ; KAWAKAMI, Y. ; HAYASHI, T. ; OHNI-SHI, M.** Simple broad-spectrum protocol using labiase for bacterial cell lysis to prepare genomic DNA for pulsed-field gel electrophoresis analysis. *Microbiology and immunology,* 2009, vol. 53 (1), 45-48
- **PHILIP, N. ; SUNEJA, B. ; WALSH, L.** Beyond Streptococcus mutans: clinical implications of the evolving dental caries aetiological paradigms and its associated microbiome. *Br Dent J,* 2018, vol. 224, 219-225, https://doi.org/10.1038/sj.bdj.2018.81
- **PITTS, N. ; ZERO, D. ; MARSH, P. et al.** Dental caries. *Nat Rev Dis Primers,* 2017, vol. 3, 17030, https://doi.org/10.1038/nrdp.2017.30
- **SIMS JR, K. R. ; MACEREN, J. P. ; LIU, Y. ; RO-CHA, G. R. ; KOO, H. ; BENOIT, D. S.** Dual antibacterial drug-loaded nanoparticles synergistically improve treatment of Streptococcus mutans biofilms. *Acta Biomaterialia,* 2020, vol. 115, 418-431
- **SCHLEIFER, K. H. ; KANDLER, O.** Peptidoglycan types of bacterial cell walls and their taxonomic implications. *Bacteriological reviews,* 1972, vol. 36 (4), 407-477
- **TANG, X. ; KUDO, Y. ; BAKER, J. L. ; LABONTE, S. ; JORDAN, P. A. ; MCKINNIE, S. M. ; EDLUND, A.** Cariogenic Streptococcus mutans produces tetramic acid strain-specific antibiotics that impair commensal colonization. *ACS infectious diseases,* 2020, vol. 6 (4), 563-571
- **TENOVUO, J.** Clinical applications of antimicrobial host proteins lactoperoxidase, lysozyme and lactoferrin in xerostomia: efficacy and safety. *Oral diseases,* 2002, vol. 8 (1), 23-29
- **YUE, J. ; YANG, H. ; LIU, S. ; SONG, F. ; GUO, J. ; HUANG, C.** Influence of naringenin on the biofilm formation of Streptococcus mutans. *Journal of dentistry,* 2018, vol. 76, 24-31
- **WILCOX, L. J. ; BORRADAILE, N. M. ; HUFF, M. W.** Antiatherogenic properties of naringenin, a citrus flavonoid. *Cardiovascular drug reviews,* 1999, vol. 17 (2), 160-178